# FASCICULE DE BREVET EUROPEEN

(11) **EP 4 205 799 B1**
(45) Date de publication et mention de la délivrance du brevet: **14.02.2024**
(21) Numéro de dépôt: 21315298.6
(22) Date de dépôt: 29.12.2021
(51) Int. Cl.: A61N 1/05, A61N 1/375

(54) **DISPOSITIF MÉDICAL IMPLANTABLE À VIS D'ANCRAGE HÉLICOÏDALE NON TRAUMATIQUE**
IMPLANTIERBARE MEDIZINISCHE VORRICHTUNG MIT ATRAUMATISCHER SPIRALFÖRMIGER VERANKERUNGSSCHRAUBE
IMPLANTABLE MEDICAL DEVICE WITH NON-TRAUMATIC HELICAL ANCHORING SCREW

(43) Date de publication de la demande: 05.07.2023
(73) Titulaire: CAIRDAC, 92160 Antony (FR)
(72) Inventeur: Nguyen-Dinh, An, 37520 La Riche (FR); Regnier, Willy, 91160 Longjumeau (FR)
(74) Mandataire: Dupuis-Latour, Dominique

(56) Documents cités:
- US-A- 4 311 153
- US-A1- 2020 197 705
- US-A1- 2020 289 835
- US-B2- 11 197 997

## Description

### Domaine de l'invention

L'invention telle que définie dans la revendication 1 concerne les dispositifs médicaux implantés, en particulier ceux de ces dispositifs qui sont destinés à être implantés dans une cavité cardiaque.

Comme on l'exposera par la suite, l'invention est de façon particulièrement avantageuse applicable à des dispositifs implantables autonomes de type "capsule *leadless",* qui sont des dispositifs implantables dépourvus de toute liaison physique (*lead*) à un dispositif distant.

L'invention n'est toutefois pas limitée à un type particulier de capsule, ni même d'implant, et elle est applicable indifféremment à de nombreux autres types de dispositifs, quelle que soit leur destination fonctionnelle, cardiaque ou autre, par exemple à des capsules destinées à diffuse *in situ* un agent pharmacologique actif.

Dans le cas d'une application cardiaque, la capsule surveille en continu le rythme du patient et délivre si nécessaire au coeur des impulsions électriques de stimulation, de resynchronisation et/ou de défibrillation en cas de trouble du rythme détecté par la capsule. La capsule comprend divers circuits électroniques, capteurs, etc., ainsi que des moyens émetteurs/récepteurs de communication sans fil pour l'échange de données à distance, le tout étant intégré dans un corps de très petites dimensions qui puisse être implanté dans des sites difficilement accessibles ou laissant peu d'espace, tels que l'apex du ventricule ou la paroi interne de l'oreillette.

En particulier, la mise en place d'une capsule auriculaire présente le très grand avantage de pouvoir mettre en oeuvre une stimulation de type "double chambre" combinant une capsule ventriculaire et une capsule auriculaire munies de moyens de communication mutuels. La capsule auriculaire détecte les dépolarisations auriculaires du rythme sinusal, et la capsule ventriculaire, ainsi qu'éventuellement la capsule auriculaire, délivre(nt) en tant que de besoin au ventricule et/ou à l'oreillette des impulsions de stimulation séquencées de manière à contrôler de façon précise le délai atrioventriculaire de stimulation.

L'invention concerne plus particulièrement l'ancrage de la capsule, ou plus généralement du dispositif médical, au site d'implantation choisi.

La capsule est munie à son extrémité distale d'un organe d'ancrage apte à pénétrer dans les tissus d'une paroi corporelle. Dans le cas d'une capsule endocavitaire, la capsule est fixée à la paroi intérieure d'une cavité ventriculaire ou auriculaire.

Il existe plusieurs types d'ancrage, souvent à base de barbes (*fines*) élastiquement déformables se déployant dans la masse du myocarde, mais aussi des ancrages par une vis hélicoïdale saillante prolongeant axialement le corps de la capsule et conçue pour pénétrer dans le tissu cardiaque au site d'implantation par vissage. L'invention concerne ce dernier type d'organe d'ancrage, à vis hélicoïdale.

La "délivrance" de la capsule, c'est-à-dire sa mise en place au site d'implantation, consiste à monter la capsule à l'extrémité d'un cathéter-guide d'un accessoire d'implantation, puis à la faire progresser le long du réseau veineux périphérique et la diriger ensuite jusqu'au site choisi, par exemple l'apex de la cavité ventriculaire droite. Une fois le site d'implantation atteint, par l'intermédiaire du cathéter-guide le praticien imprime à la capsule un mouvement combiné de translation axiale (pour faire progresser la capsule vers l'avant puis exercer une pression contre la paroi cardiaque) et de rotation de la capsule sur elle-même (pour visser l'organe d'ancrage dans l'épaisseur de la paroi cardiaque). Une fois la capsule fermement ancrée-, le praticien procède au "largage" de la capsule, c'est-à-dire à sa désolidarisation d'avec l'accessoire d'implantation, la capsule devenant alors totalement autonome.

### Description de la technique antérieure

Le WO 2002/064172 A1 (Cairdac) (ou US 11 197 997 B2 (Regnier)), décrit une capsule munie d'une vis d'ancrage hélicoïdale, et pourvue en outre d'un système de limitation de couple permettant de débrayer le corps de la capsule de la vis d'ancrage lorsque le couple de réaction exercé par la vis d'ancrage dépasse un seuil prédéterminé, de manière à ne pas dépasser une valeur limite dite "coupe de carottage" au-delà de laquelle la vis d'ancrage risquerait de déchirer localement les tissus sous l'effet de la rotation de la vis sans avance de celle-ci, jusqu'à provoquer une lacération des tissus et, à l'extrême, une perforation de la paroi avec risque de tamponnade.

Comme on l'expliquera en détail par la suite, ce risque d'endommagement des tissus est particulièrement élevé dans le cas d'une implantation dans une paroi mince telle que celle de l'oreillette, notamment dans la région de l'appendice auriculaire droit ou RAA (*Right Atrial Appendage*), compte tenu de l'épaisseur très faible, typiquement de l'ordre de 0,5 à 2 mm suivant l'emplacement. La difficulté est accrue par le fait que la zone du RAA contient de nombreux replis et qu'il n'est pas possible de prédire l'épaisseur exacte au site d'implantation avant le vissage et que de plus, en cas de perforation de la paroi cardiaque, il n'est pas possible de déceler un saignement, l'épanchement du sang se produisant à l'intérieur du sac péricardique (tamponnage hémorragique). De plus, du fait de ces replis, il n'est pas possible de déterminer exactement l'angle que formera la capsule avec le myocarde au moment du contact avec la paroi cardiaque. Si la direction est oblique, le risque de perforation est moindre mais en revanche le risque de mauvais seuil de détection sera accru, et inversement.

En tout état de cause, compte tenu de l'épaisseur réduite de la paroi, la longueur fixante de l'hélice (longueur de la vis au-delà de la face plane frontale de la capsule supportant l'électrode de détection/stimulation) devra nécessairement être inférieure à 1,5 mm, qui est une longueur réduite qui rend difficile l'obtention d'un ancrage fiable et durable du dispositif à la paroi de l'oreillette.

Le but de la présente invention est de proposer un dispositif médical implantable muni d'un ancrage à vis permettant une fixation mécaniquement sûre et cliniquement sans risque à une paroi mince, en faisant en sorte de ne pas traverser la paroi mince de l'organe avec l'hélice - notamment, dans le cas d'une implantation dans l'oreillette, de préserver l'espace péricardique et prévenir tout saignement. Le document US 2020/289835 A1 (EBY THOMAS B) divulgue un dispositif selon le préambule de la revendication 1.

### RÉSUMÉ DE L'INVENTION

Pour résoudre ces problèmes et atteindre les buts exposés ci-dessus, l'invention propose à cet effet un dispositif médical implantable selon la revendication 1.

Diverses caractéristiques subsidiaires avantageuses sont précisées dans les revendications dépendantes 2 à 10.

Le dispositif de l'invention peut notamment être un implant cardiaque autonome de type capsule *leadless,* dans lequel le corps de dispositif loge un ensemble électronique et un module de récupération d'énergie avec un moyen de stockage d'énergie pour l'alimentation de l'ensemble électronique, et dans lequel la face frontale du corps de dispositif porte une électrode de détection/stimulation cardiaque apte à venir en contact avec la paroi de l'organe du patient après ancrage du dispositif.

### DESCRIPTION SOMMAIRE DES DESSINS

On va maintenant décrire un exemple de réalisation de la présente invention en référence aux dessins annexés, où les mêmes références désignent d'une figure à l'autre des éléments identiques ou fonctionnellement semblables.
La Figure 1 illustre des dispositifs médicaux de type capsule *leadless* dans leur environnement, avec divers exemples de sites d'implantation dans, sur ou à proximité du coeur d'un patient.
La Figure 2 est une vue d'ensemble extérieure d'une capsule *leadless,* montrant notamment la vis d'ancrage et l'électrode de stimulation à son extrémité distale.
La Figure 3 montre plus précisément l'extrémité distale d'une capsule *leadless* munie d'une vis d'ancrage selon l'état de la technique, avec l'extrémité de la vis à l'approche de la paroi du site d'implantation.
La Figure 4 est une vue de détail de l'extrémité biseautée de la vis d'ancrage de la Figure 3.
La Figure 5 est une vue en coupe d'une paroi cardiaque, notamment au niveau de l'oreillette, détaillant les différents tissus qui constituent cette paroi.
La Figure 6 est une vue en perspective d'une vis d'ancrage selon la présente invention.
La Figure 7 est un détail de l'extrémité distale de la vis d'ancrage de la Figure 6.
La Figure 8 illustre une variante de la Figure 7.
La Figure 9 illustre un mode de réalisation particulier où la vis d'ancrage présente une raideur en flexion qui n'est pas constante au voisinage de l'extrémité distale de l'hélice.
La Figure 10 montre l'extrémité distale d'une vis d'ancrage selon l'invention, pendant la pénétration de la paroi cardiaque au site d'implantation.
La Figure 11 est une vue schématique agrandie de l'extrémité biseautée de la vis d'ancrage selon l'invention, explicitant les différentes forces exercées au niveau de cette extrémité, ainsi que leur résultante.

### DESCRIPTION DÉTAILLÉE DE MODES DE RÉALISATION PRÉFÉRENTIELS DE L'INVENTION

On va maintenant décrire un exemple de réalisation du dispositif de l'invention, dans une application à une capsule implantable autonome destinée à être implantée dans une cavité cardiaque.

Comme on l'a indiqué plus haut, cette application particulière n'est donnée qu'à titre d'exemple de réalisation et n'est pas limitative de l'invention, dont les enseignements peuvent être appliqués à de nombreux autre types de dispositifs implantables.

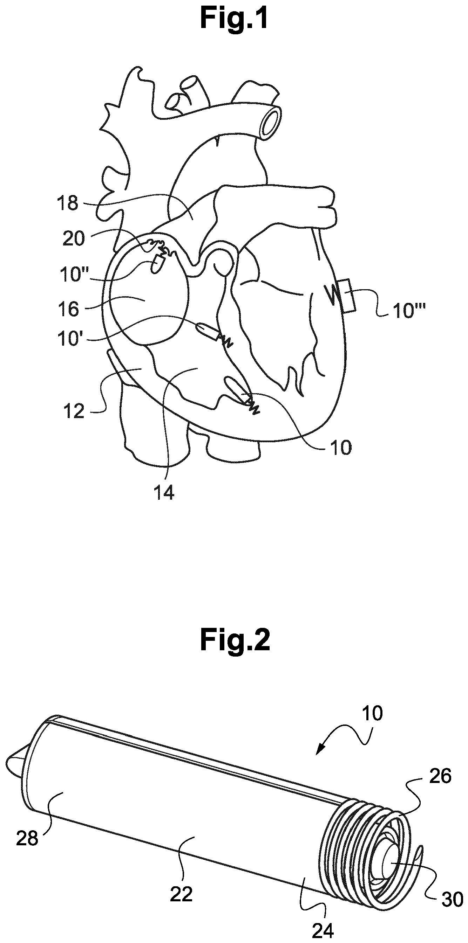

Sur la Figure 1, on a représenté diverses possibilités de sites d'implantation d'un dispositif médical implantable de type capsule *leadless,* dans une application à la stimulation cardiaque. Ainsi, la capsule référencée 10 est implantée à l'apex du ventricule droit 14 du myocarde 12. La capsule peut être également implantée sur le septum interventriculaire, comme en 10', ou encore sur une paroi de l'oreillette droite 16, comme en 10". Le dispositif peut également être une capsule épicardique placée sur une région externe du myocarde, comme illustré en 10'".

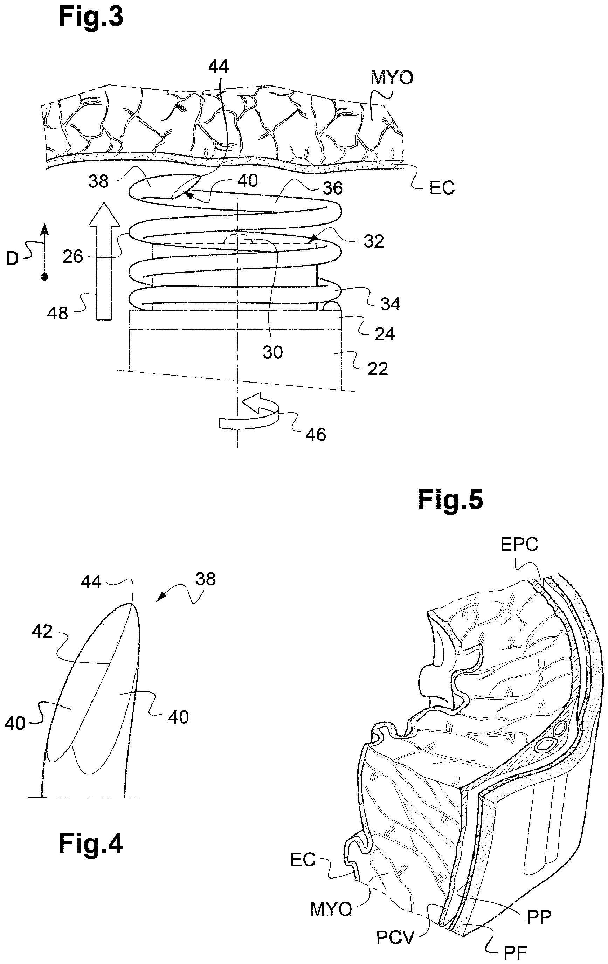

On décrira plus en détail par la suite, en référence notamment à la Figure 5, le cas spécifique d'une implantation dans la région de l'appendice auriculaire droit 18, où la paroi cardiaque à l'intérieur de l'oreillette 16 présente une épaisseur plus réduite, ainsi qu'un certain nombre de replis et trabéculations 20 ; cette région présente l'avantage de ne pas se situer dans le prolongement de la veine cave supérieure et de la valve tricuspide, de sorte que la présence d'une capsule à cette endroit ne gênera pas l'accès aux autres zones des cavités auriculaire ou ventriculaire, en particulier pour accéder au fond du ventricule si l'on veut implanter ou explanter une capsule dans la région de l'apex.

Dans tous les cas, la capsule *leadless* est fixée à la paroi cardiaque au moyen d'un système d'ancrage saillant pénétrant dans le tissu cardiaque pour la maintenir en place au site d'implantation. Divers systèmes sont utilisables, l'invention concernant plus particulièrement les capsules munies d'un organe d'ancrage à vis hélicoïdale.

Comme illustré sur la Figure 2, une capsule munie d'un tel organe se présente globalement sous la forme d'un corps tubulaire 22 enfermant les divers circuits électroniques et d'alimentation de la capsule. Les dimensions typiques des capsules connues sont un diamètre de l'ordre de 6 mm pour une longueur d'environ 25 à 40 mm. Le corps tubulaire 22 comporte à son extrémité avant (distale) 24 une vis hélicoïdale cylindrique 26 destinée à maintenir la capsule en place au site d'implantation. L'extrémité arrière opposée (proximale) 28 est une extrémité libre, qui est seulement pourvue de moyens (non représentés) de liaison temporaire à un cathéter-guide ou autre accessoire d'implantation utilisable pour la mise en place ou l'explantation de la capsule et qui est ensuite désolidarisé de cette dernière.

Pour assurer les fonctions de détection/stimulation, une électrode 30 est disposée sur la face frontale de la capsule ; une fois la capsule ancrée au site d'implantation, l'électrode 30 est en contact avec le tissu cardiaque, permettant ainsi le recueil des potentiels de dépolarisation et/ou l'application d'impulsions de stimulation.

La Figure 3 montre plus précisément l'extrémité distale de la capsule avec la vis hélicoïdale 26 à l'approche de la paroi cardiaque, comprenant le myocarde MYO et l'endocarde EC.

Au moment de l'implantation, la face frontale 32 du corps 22 de la capsule, qui porte l'électrode 30, est tournée vers la paroi de l'endocarde. La vis hélicoïdale 26 comprend, côté proximal, des spires inactives 34 solidaires du corps 22 de la capsule et, à l'opposé, des spires actives 36 terminées par une extrémité libre biseautée 38.

Une telle capsule est par exemple décrite par le WO 2002/064172 A1 (US 11 197 997 B2) précité.

La Figure 4 illustre plus précisément la pointe biseautée 38, qui est une pointe perforante comprenant une ou plusieurs surfaces obliques 40 permettant de créer une arête coupante 42 avec une pointe perforante 44 à l'extrémité la plus distale de la vis hélicoïdale. Comme on peut le voir sur la Figure 3, l'extrémité perforante 44 est dirigée vers la paroi cardiaque, avec les surfaces obliques 40 tournées vers la direction proximale (la direction distale étant indiquée en D sur la Figure 3).

Cette configuration avec la pointe tournée vers la paroi (c'est-à-dire orientée en direction distale D) facilite la perforation de l'endocarde EC puis la pénétration dans le myocarde MYO dans les différentes configurations susceptibles d'être rencontrées, même en cas d'inclinaison entre l'axe de la capsule et la normale à la paroi au moment de la venue en contact avec cette dernière. Une fois la pointe 44 en contact avec l'endocarde EC, le mouvement combiné de rotation 46 et de translation 48 (poussée axiale) exercé par le praticien via le cathéter-guide assure la perforation de l'endocarde EC et la pénétration de la vis dans le myocarde MYO sur la longueur des spires actives 36, qui est de l'ordre de 1,5 mm ou plus pour une fixation dans la cavité ventriculaire. La face frontale 32 portant l'électrode 30 est alors en contact avec la paroi cardiaque, permettant à l'électrode d'assurer ses fonctions de détection/stimulation.

La configuration connue de vis d'ancrage que l'on vient de décrire en référence aux Figures 3 et 4 est toutefois mal adaptée à une fixation dans l'oreillette, car il faut prendre garde à ne pas perforer la paroi cardiaque. La Figure 5 illustre les différents tissus constituant la paroi cardiaque de l'oreillette, notamment au niveau de l'appendice auriculaire droit (RAA) : la paroi comprend, de l'intérieur vers l'extérieur, l'endocarde EC, le myocarde MYO, le péricarde viscéral PCV, l'espace péricardique EPC, le péricarde pariétal PP et le péricarde fibreux PF.

L'épaisseur totale de la paroi est comprise entre 0,5 et 2 mm, donc une valeur beaucoup plus faible que celle du ventricule, que ce soit au niveau de l'apex ou du septum. De plus, le péricarde viscéral PCV est une paroi très fine, d'épaisseur environ 0,2 mm, qui est traversée par endroits par des vaisseaux sanguins coronaires. Si l'on veut implanter dans une telle paroi une capsule munie d'un organe d'ancrage à vis, il ne faut surtout pas traverser le péricarde viscéral PCV avec l'hélice de la vis, afin d'éviter toute perforation qui créerait un risque de tamponnade hémorragique avec épanchement de sang dans l'espace péricardique EPC. Cela étant, il est nécessaire de pénétrer suffisamment le myocarde MYO pour garantir une bonne fixation de la capsule à la paroi cardiaque.

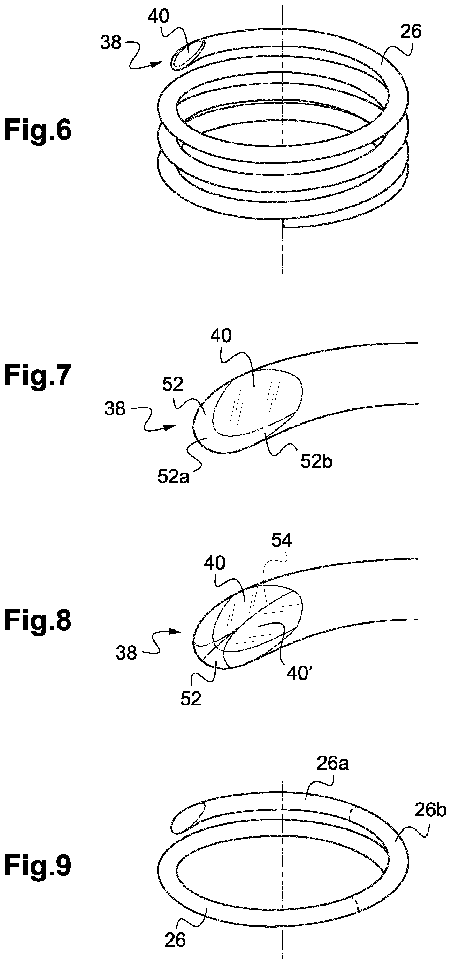

Pour résoudre ce problème, l'invention propose une nouvelle configuration de vis d'ancrage, illustrée aux Figures 6 à 11.

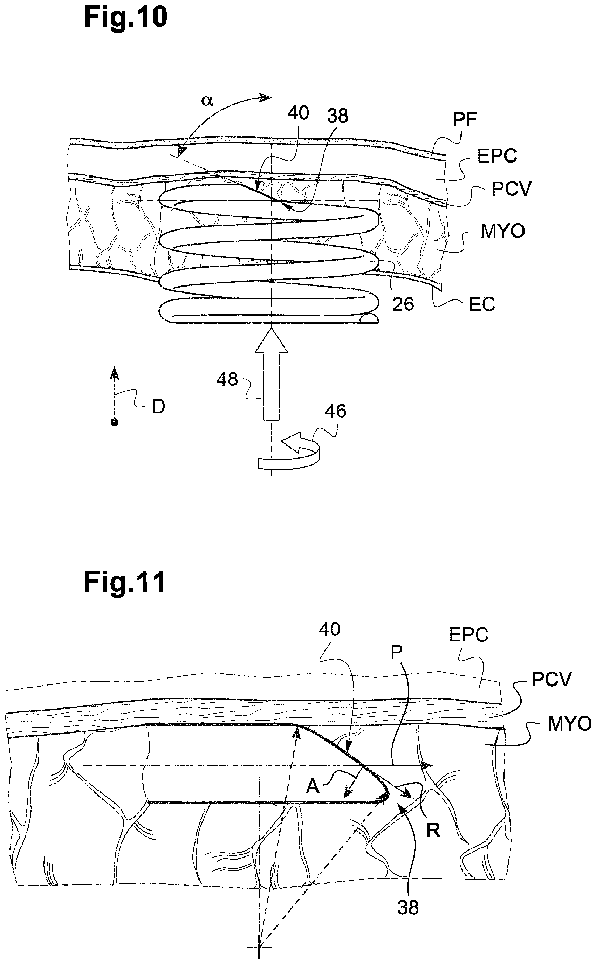

Comme illustré Figure 6, la vis hélicoïdale 26 est terminée par une extrémité libre biseautée 38 définie par au moins une surface oblique 40. La surface oblique 40 peut être une surface plane. Elle peut également être une surface courbe, par exemple une surface conique telle que la surface d'un cône de révolution ou d'un cône oblique dont la région voisine du sommet aurait été émoussée pour ne pas être traumatique. De façon caractéristique de l'invention, la surface oblique 40 définissant le biseau de l'extrémité 38 est tournée en direction de la paroi, c'est-à-dire en direction distale D (cf. Figure 10). En d'autres termes, la normale à la surface oblique 40 est orientée dans une direction opposée à la face frontale du corps du dispositif, de manière que cette surface oblique 40 soit tournée vers la paroi de l'organe - à la différence des vis d'ancrage de l'état de la technique, où la surface oblique du biseau est tournée dans une direction opposée à la paroi (comme illustré Figure 3).

La normale à la surface oblique forme un angle α (cf. Figure 10) compris typiquement entre 30° et 60° par rapport à l'axe de l'hélice.

Une autre caractéristique de la vis hélicoïdale d'ancrage selon l'invention est qu'elle est élastiquement déformable en compression axiale, avec un coefficient de raideur d'au plus 5 N/mm.

Le fil d'hélice peut notamment présenter les caractéristiques suivantes (qui sont en elles-mêmes non limitatives de l'invention) :
- enroulement à droite ;
- matériau biocompatible tel qu'acier inoxydable, titane, nitinol, etc., avantageusement avec un revêtement procurant un état de surface formant des microreliefs (revêtement de nitrure de titane) ou une porosité (par attaque chimique) qui favorisent après implantation l'adhésion de la vis à des tissus fibrotiques ;
- diamètre du fil : 0,2 à 1 mm ;
- diamètre extérieur de l'hélice : inférieur à 26 French (8,67 mm) ;
- nombre spires actives : 1 à 5 spires ;
- pas d'enroulement à vide : 1 mm (le pas pouvant éventuellement être un pas variable) ;
- longueur des spires actives en direction axiale : inférieure à 1,5 mm.

L'extrémité biseauté 38 est réalisée par un usinage permettant de façonner la surface oblique 40. Avantageusement, comme illustré Figure 7, un arrondi 52 est confectionné sur le pourtour de la surface oblique 40, par usinage traditionnel ou par des tirs laser. Le bord arrondi 52 permet de supprimer l'arête coupante (que l'on avait avec les vis hélicoïdales de l'art antérieur telles que celles illustrées Figures 3 et 4) et de rendre atraumatique la géométrie de l'extrémité de la vis hélicoïdale. On prévient ainsi la perforation du péricarde viscéral en supprimant toute pointe ou arête coupante à l'extrémité de l'hélice. Avantageusement, l'arrondi formé à l'extrémité de l'hélice est un arrondi variable, avec une valeur de rayon diminuant progressivement depuis l'extrémité distale 52a jusqu'à la région la moins distale 52b. Le diamètre de l'arrondi à l'extrémité distale 52a est par exemple de 0,1 mm, de façon générale un diamètre d'arrondi inférieur à 1/5 du diamètre du fil.

La Figure 8 illustre une variante de la Figure 7, dans laquelle l'extrémité biseautée 38 comprend deux surfaces obliques 40, 40' formant à leur intersection une arête 54, également arrondie de manière à n'être pas coupante.

La Figure 9 illustre un mode de réalisation particulier dans lequel la raideur en flexion du fil d'hélice (raideur du fil sur le développé de l'hélice, à distinguer de la raideur en compression axiale évoquée plus haut) est une raideur variable. À cet effet, le fil est constitué d'une ou plusieurs zones ayant des raideurs en flexion différentes, par exemple une juxtaposition de portions de fil 26a, 26b de raideurs respectives K1 et K2 différentes, ou avec un fil de diamètre variable, ou encore par un mode de fabrication spécifique (recuit thermique différencié selon les régions du fil). Des valeurs de raideur, non limitatives, sont par exemple K1 = 0,3 N/mm, K2 = 0,6 N/mm, ou bien K variable de 0,1 à 1 N/mm, sur une longueur de la demi-spire terminale de l'hélice.

On va maintenant expliquer, en référence aux Figures 10 et 11, la manière dont est opérée l'implantation de la capsule au moyen de la vis selon l'invention que l'on vient de décrire.

Avec cette vis, à la différence des solutions de l'art antérieur, l'extrémité de la vis hélicoïdale est conçue de façon à ne pas perforer la paroi cardiaque.

Il s'agit essentiellement, sous l'effet du double mouvement de rotation 46 et de compression axiale 48 imprimé par l'opérateur, de faire pénétrer l'hélice dans le myocarde sans couper celui-ci (qui est très peu résistant), puis faire en sorte que l'extrémité de l'hélice vienne glisser sur le péricarde viscéral PCV sans le transpercer, créant ainsi un mode de fixation non perforant. La surface oblique inclinée 40 viendra seulement repousser le péricarde viscéral en glissant dessus, sans le percer.

La Figure 11 montre la répartition des efforts et des contraintes à l'extrémité de l'hélice.

Dans une phase initiale, lorsque l'hélice a pénétré dans le myocarde et n'a pas encore atteint le péricarde viscéral, la force d'appui axial sur le tissu cardiaque est inférieure à la raideur en compression axiale K du fil d'hélice. La force d'appui A de la surface oblique 40, combinée à la poussée radiale P produite par la rotation de la vis, produit à l'extrémité 38 du biseau une résultante R insuffisante pour traverser le péricarde viscéral PCV et risquer de l'endommager.

Une fois que la vis a atteint le péricarde viscéral PCV, la force d'appui devient supérieure à la raideur K du fil d'hélice, du fait que la résistance du péricarde viscéral PCV est plus élevée que celle du myocarde MYO. L'hélice de la vis va alors se comprimer, en écrasant le myocarde sans perforer le péricarde viscéral.

De plus, si l'on prévoit comme exposé plus haut en référence à la Figure 9 un fil d'hélice présentant une raideur en flexion variable, une fois atteint le péricarde viscéral la partie terminale de la vis se déformera dans une géométrie non hélicoïdale du fait de la plus grande souplesse (moindre raideur à la flexion) de son extrémité.

Avantageusement, l'implant est muni d'un limiteur de couple, réalisé par exemple selon les enseignements du WO 2002/064172 A1 (US 11 197 997 B2) précité, qui permettra de débrayer l'organe d'ancrage d'avec le corps de l'implant lorsque le couple de réaction exercé par la vis dans la paroi de l'organe dépasse un couple seuil prédéterminé, évitant ainsi toute lacération des tissus.

La configuration particulière de vis hélicoïdale que l'on vient de décrire permet de disposer d'une hélice d'ancrage "intelligente", adaptative : dans le tissu fragile mais plus résistant du péricarde, le risque de perforation est fortement réduit, en gardant dans le tissu plus mou du myocarde une capacité de glissement. La longueur fixante de l'hélice est ainsi maximisée sur la quasi-totalité de l'épaisseur de la paroi de l'oreillette, bien que celle-ci soit plus mince et plus fragile que dans le cas d'une implantation ventriculaire.

## Revendications

1. Un dispositif médical implantable (10) apte à être implanté dans une paroi cardiaque mince d'un patient, notamment dans la paroi auriculaire (12) comprenant successivement un endocarde (EC), un myocarde (MYO) et un péricarde viscéral (PCV), le dispositif comprenant :
- un corps de dispositif (22) avec une face frontale (32) à son extrémité distale ; et
- un moyen d'ancrage du dispositif médical à ladite paroi cardiaque (EC, MYO, PCV),
le moyen d'ancrage comportant une vis (26) avec un fil d'hélice enroulé en une pluralité de spires non jointives, la vis ayant une extrémité encastrée (34) solidarisée à la face frontale (32) du corps de dispositif (22) et une extrémité libre (36) comprenant une extrémité biseautée (38) définie par au moins une surface oblique (40), dans lequel :
- la normale à la surface oblique (40) de l'extrémité biseautée (38) est orientée dans une direction opposée à la face frontale (32) du corps de dispositif (22), de sorte que la surface oblique (40) soit orientée vers la paroi cardiaque (EC, MYO, PCV),
- la normale à la surface oblique (40) forme un angle (α) compris entre 30° et 60° par rapport à l'axe de l'hélice, et
- la vis (26) est élastiquement déformable en compression axiale, ***caractérisé en ce que*** la vis est une vis non perforante dépourvue de pointe ou arête coupante, dans laquelle :
- la surface oblique (40) comporte à son extrémité un bord arrondi (52) apte à glisser sur le péricarde viscéral (PCV) sans le transpercer,
- la longueur des spires actives de la vis (26) est inférieure à 1,5 mm, et le coefficient K de raideur de la vis en compression axiale est d'au plus 5 N/mm, tel que, lors d'une implantation dans une paroi auriculaire, la vis, une fois atteint le péricarde viscéral (PCV), produise un écrasement du myocarde (MYO) sans perforation du péricarde viscéral (PCV),
de manière à prévenir ainsi tout épanchement de sang dans l'espace péricardique (EPC).

2. Le dispositif de la revendication 1, dans lequel la surface oblique (40) est une surface plane.

3. Le dispositif de la revendication 1, dans lequel le rayon de courbure du bord arrondi (52) de la surface oblique (40) est tel que le ratio diamètre d'arrondi : diamètre du fil soit d'au plus 1:5.

4. Le dispositif de la revendication 3, dans lequel le rayon de courbure du bord arrondi (52) est un rayon progressivement décroissant depuis une valeur maximale à l'endroit (52a) de l'extrémité la plus distale du bord de la surface oblique (40).

5. Le dispositif de la revendication 1, dans lequel l'extrémité biseautée (38) comprend deux surfaces obliques (40, 40') mutuellement agencées en un dièdre, et dans lequel la normale à chaque surface oblique (40) est orientée dans une direction opposée à la face frontale (32) du corps de dispositif (22), de sorte que les deux surfaces obliques (40, 40') ainsi qu'une arête (54) du dièdre soient orientées vers la paroi (EC) de l'organe (12) du patient.

6. Le dispositif de la revendication 1, dans lequel le coefficient K de raideur de la vis (26) en compression axiale est d'au plus 1 N/mm.

7. Le dispositif de la revendication 1, comprenant en outre un couplage débrayable agencé pour, lorsqu'une sollicitation externe en rotation axiale est appliquée au corps de dispositif (22) dans un sens de vissage de la vis (26), autoriser une rotation relative axiale entre le corps de dispositif (22) et le moyen d'ancrage dès qu'un couple de réaction exercé par la vis (26) dans la paroi (EC) de l'organe (12) du patient dépasse un couple seuil prédéterminé.

8. Le dispositif de la revendication 1, dans lequel, au moins sur l'étendue d'une demi-spire terminale de l'hélice, la raideur en flexion du fil d'hélice est une raideur non constante, plus faible dans une région (26) située au voisinage de l'extrémité libre (36) du fil d'hélice.

9. Le dispositif de la revendication 1, dans lequel le fil d'hélice de la vis (26) présente au moins l'une des caractéristiques suivantes :
- diamètre du fil : 0,2 à 1 mm ;
- diamètre extérieur de l'hélice : inférieur à 26 Fr (8,67 mm) ;
- nombre de spires : 1 à 5 spires ;
- pas d'enroulement à vide : 1 mm, constant ou variable.

10. Le dispositif de la revendication 1, dans lequel :
- le dispositif médical est un implant cardiaque autonome de type capsule *leadless,*
- le corps de dispositif (22) loge un ensemble électronique et un module de récupération d'énergie avec un moyen de stockage d'énergie pour l'alimentation de l'ensemble électronique, et
- la face frontale (32) du corps de dispositif (22) porte une électrode (30) de détection/stimulation cardiaque apte à venir en contact avec la paroi (EC) de l'organe (12) du patient après ancrage du dispositif.

## Patentansprüche

1. Implantierbare medizinische Vorrichtung (10), die eingerichtet ist, in einer dünnen Herzwand eines Patienten implantiert zu werden, insbesondere in der Vorhofwand (12), die nacheinander ein Epikard (EC), ein Myokard (MYO) und ein viszerales Perikard (PCV) umfasst, wobei die Vorrichtung umfasst:
- einen Vorrichtungskörper (22) mit einer Stirnfläche (32) an seinem distalen Ende; und
- ein Mittel zur Verankerung der medizinischen
Vorrichtung an der Herzwand (EC, MYO, PCV), wobei das Verankerungsmittel eine Schraube (26) mit einem Spiraldraht aufweist, der in einer Mehrzahl von nicht aneinander liegenden Windungen gewickelt ist, wobei die Schraube ein eingestecktes Ende (34) aufweist, das mit der Stirnfläche (32) des Vorrichtungskörpers (22) fest verbunden ist, und ein freies Ende (36) aufweist, das ein abgefastes Ende (38) umfasst, das von wenigstens einer schrägen Oberfläche (40) definiert ist, wobei:
- die Normale zu der schrägen Oberfläche (40) des abgefasten Endes (38) in einer Richtung gerichtet ist, die zu der Stirnfläche (32) des Vorrichtungskörpers (22) entgegengesetzt ist, so dass die schräge Oberfläche (40) zu der Herzwand (EC, MYO, PVC) hin gerichtet ist,
- die Normale zu der schrägen Oberfläche (40) einen Winkel (α) zwischen 30° und 60° relativ zu der Achse der Spirale bildet und
- die Schraube (26) in axialer Stauchung elastisch verformbar ist,
***dadurch gekennzeichnet, dass*** die Schraube eine nicht lochformende Schraube ohne Spitze oder Schneidkante ist, wobei:
- die schräge Fläche (40) an ihrem Ende eine gerundete Kante (52) aufweist, die eingerichtet ist, um auf dem viszeralen Perikard (PCV) zu gleiten, ohne ihn zu durchbohren,
- die Länge der federnden Windungen der Schraube (26) kleiner als 1,5 mm ist und der Steifigkeitskoeffizient K der Schraube in axialer Stauchung höchstens 5 N/mm beträgt, dergestalt, dass, bei einer Implantation in einer Vorhofwand, die Schraube, sobald sie das viszerale Perikard (PCV) erreicht, eine Stauchverformung des Myokard (MYO) ohne Durchbohrung des viszeralen Perikard (PCV) bewirkt,
so dass so jedwede Ansammlung von Blut in dem Herzbeutel (EPC) verhindert wird.

2. Vorrichtung nach Anspruch 1, wobei die schräge Oberfläche (40) eine ebene Oberfläche ist.

3. Vorrichtung nach Anspruch 1, wobei der Krümmungsradius der gerundeten Kante (52) der schrägen Oberfläche (40) dergestalt ist, dass das Verhältnis von Rundungsdurchmesser zu Drahtdurchmesser höchstens 1:5 beträgt.

4. Vorrichtung nach Anspruch 3, wobei der Krümmungsradius der gerundeten Kante (52) ein Radius ist, der von einem Höchstwert an der Stelle (52a) des distalsten Endes des Randes der schrägen Oberfläche (40) progressiv abnimmt.

5. Vorrichtung nach Anspruch 1, wobei das abgefaste Ende (38) zwei schräge Oberflächen (40, 40') umfasst, die in einer V-Form zueinander angeordnet sind, und wobei die Normale zu jeder schrägen Oberfläche (40) in einer Richtung gerichtet ist, die zu der Stirnfläche (32) des Vorrichtungskörpers (22) entgegengesetzt ist, so dass die zwei schrägen Oberflächen (40, 40') sowie eine Kante (54) der V-Form zu der Wand (EC) des Organs (12) des Patienten gerichtet sind.

6. Vorrichtung nach Anspruch 1, wobei der Steifigkeitskoeffizient K der Schraube (26) in axialer Stauchung höchstens 1 N/mm beträgt.

7. Vorrichtung nach Anspruch 1, ferner umfassend eine auskuppelbare Kupplung, die angeordnet ist, um, wenn eine äußere axiale Rotationsbelastung auf den Vorrichtungskörper (22) in einer Verschraubungsrichtung der Schraube (26) aufgebracht wird, eine axiale relative Rotation zwischen dem Vorrichtungskörper (22) und dem Verankerungsmittel zu gestatten, sobald ein Reaktionsmoment, das von der Schraube (26) in der Wand (EC) des Organs (12) des Patienten ausgeübt wird, einen vorbestimmten Schwellwert übersteigt.

8. Vorrichtung nach Anspruch 1, wobei, wenigstens über die Ausdehnung einer halben Endwindung der Spirale, die Biegesteifigkeit des Spiraldrahtes eine nicht konstante Steifigkeit ist, die in einer Region (26), die sich in der Nähe des freien Endes (36) des Spiraldrahtes befindet, geringer ist.

9. Vorrichtung nach Anspruch 1, wobei der Spiraldraht der Schraube (26) wenigstens eines der folgenden Merkmale aufweist:
- Durchmesser des Drahtes: 0,2 bis 1 mm;
- äußerer Durchmesser der Spirale: kleiner als 26 French (8,67 mm);
- Anzahl an Windungen: 1 bis 5 Windungen;
- Windungshöhe unbelastet: 1 mm, konstant oder variabel.

10. Vorrichtung nach Anspruch 1, wobei:
- die medizinische Vorrichtung ein autonomes Herzimplantat vom Typ leadless-Kapsel ist,
- der Vorrichtungskörper (22) eine elektronische Anordnung und ein Energierückgewinnungsmodul mit einem Energiespeichermittel für die Versorgung der elektronischen Anordnung aufnimmt und
- die Stirnseite (32) des Vorrichtungskörpers (22) eine Elektrode (30) zur Erfassung/Stimulation des Herzschlags trägt, die dazu eingerichtet ist, nach Verankerung der Vorrichtung mit der Wand (EC) des Organs (12) des Patienten in Kontakt zu kommen.

## Claims

1. An implantable medical device (10) adapted to be implanted into a thin heart wall of a patient, in particular into an atrial wall (12) comprising successively an endocardium (EC), a myocardium (MYO) and a visceral pericardium (PCV), the device comprising:
- a device body (22) with a front face (32) at its distal end; and
- a means for anchoring the medical device to said heart wall (EC, MYO, PCV),
wherein the means for anchoring includes a screw (26) with a helix wire wound into a plurality of non-contiguous turns, the screw having a clamped end (34) integral with the front face (32) of the device body (22) and a free end (36) comprising a beveled end (38) defined by at least one oblique surface (40), wherein:
- the normal to the oblique surface (40) of the beveled end (38) is directed away from the front face (32) of the device body (22), in such a way that the oblique surface (40) is directed towards the heart wall (EC, MYO, PCV),
- the normal to the oblique surface (40) forms an angle (α) between 30° and 60° with respect to the helix axis, and
- the screw (26) is elastically deformable in axial compression,
***characterised in that*** the screw is a non-piercing screw devoid of tip or cutting edge, wherein:
- the oblique surface (40) has at its end a rounded edge (52) adapted to slide over the visceral pericardium(PCV) without piercing it,
- the length of the active turns of the screw (26) is lower than 1.5 mm, and the stiffness coefficient K of the screw in axial compression is at most 5 N/mm, so that, during an implantation in an atrial wall, the screw, after having reached the visceral pericardium (PCV), produces a crushing of the myocardium (MYO) without piercing the visceral pericardium (PCV),
whereby avoiding any blood effusion in the pericardial space (EPC).

2. The device of claim 1, wherein the oblique surface (40) is a flat surface.

3. The device of claim 1, wherein the radius of curvature of the rounded edge (52) of the oblique surface (40) is such that the rounded diameter: wire diameter ratio is at most 1:5.

4. The device of claim 3, wherein the radius of curvature of the rounded edge (52) is a radius which progressively decreases from a maximum value at the most distal end (52a) of the oblique surface (40).

5. The device of claim 1, wherein the beveled end (38) has two oblique surfaces (40, 40') mutually arranged as a dihedron, and wherein the normal to each oblique surface (40) is directed away form the front face (32) of the device body (22), in such a way that the two oblique surfaces (40, 40') as well as an edge (24) of the dihedron are directed towards the wall (EC) of the patient's organ (12).

6. The device of claim 1, wherein the stiffness coefficient K of the screw in axial compression is at most 1 N/mm.

7. The device of claim 1, further comprising a disengageable coupling arranged in such a way as, when an external axial rotational stress is applied to the device body (22) in a screwing direction of the screw (26), to allow a relative axial rotation between the device body (22) and the means for anchoring as soon as a reaction torque exerted by the screw (26) in the wall (EC) of the patient's organ (12) exceeds a predetermined threshold torque.

8. The device of claim 1, wherein, at least over the extent of a terminal half-turn of the helix, the bending stiffness of the helix wire is a non-constant stiffness, lower in a region (26) located near the free end (36) of the helix.

9. The device of claim 1, wherein the helix wire of the screw (26) has at least one of the following characteristics:
- wire diameter: 0.2 to 1 mm;
- external diameter of the helix: lower than 26 Fr (8.67 mm);
- number of turns: 1 to 5 turns;
- winding pitch under zero-load condition: 1 mm, constant or variable.

10. The device of claim 1, wherein:
- the medical device is an autonomous cardiac implant of the leadless capsule type,
- the device body (22) houses an electronic unit and an energy harvesting module with an energy storage means for powering the electronic unit, and
- the front face (32) of the device body (22) carries a cardiac sensing/pacing electrode (30) adapted to come into contact with the wall (EC) of the patient's organ (12) after anchoring of the device.
